# EUROPEAN PATENT SPECIFICATION

(11) **EP 3 052 171 B1**
(45) Date of publication and mention of the grant of the patent: **26.12.2018**
(21) Application number: 14793617.3
(22) Date of filing: 02.10.2014
(51) Int. Cl.: A61M 16/04

(54) **ARTIFICIAL AIRWAY DEVICE**
KÜNSTLICHE ATEMWEGSVORRICHTUNG
DISPOSITIF DE RESPIRATION ARTIFICIELLE

(30) Priority: 04.10.2013 GB 201317596
(43) Date of publication of application: 10.08.2016
(60) Divisional application: 18205093.0
(73) Proprietor: Teleflex Life Sciences Unlimited Company, Hamilton, HM 08 (BM)
(72) Inventor: POULSEN, Sylwia, 018980 Singapore (SG); KHEONG, Ooi Wee, 11700 Penang (MY)
(74) Representative: Jacob, Reuben Ellis
(86) International application number: PCT/IB2014/002140
(87) International publication number: WO 2015/049582

(56) References cited:
- EP-A2- 0 982 045
- GB-A- 2 344 766
- US-A- 4 344 428
- US-A- 4 351 331
- US-A- 5 513 627
- US-A- 5 643 174
- US-A- 5 699 787
- US-A- 6 119 695
- US-A1- 2003 051 734
- US-A1- 2003 094 177
- US-B1- 6 318 367

## Description

The invention relates to an artificial airway device to facilitate lung ventilation in a patient.

Artificial airway devices such as endotracheal tubes and laryngeal mask airway devices are well known devices useful for establishing airways in patients. An endotracheal tube is a flexible tube of rubber or plastics material, usually with an inflatable cuff around its distal end and intended for introduction through the mouth and larynx into the trachea whereupon the cuff is inflated to seal against the wall of the trachea. In its most basic form a laryngeal mask airway device consists of an airway tube and a mask carried at one end of the airway tube, the mask having a peripheral formation often known as a "cuff' which is capable of conforming to and of fitting within, the actual and potential space behind the larynx of the patient so as to form a seal around the laryngeal inlet. The cuff can be inflatable, and in most variants it surrounds a hollow interior space or lumen of the mask, the at least one airway tube opening into the lumen. It is relatively easy to insert a laryngeal mask airway device into a patient and thereby establish an airway. Also, the laryngeal mask airway device is a "forgiving" device in that even if it is inserted improperly, it still tends to establish an airway. Accordingly, the laryngeal mask airway device is often thought of as a "life saving" device. Also, the laryngeal mask airway device may be inserted with only relatively minor manipulation of the patient's head, neck and jaw. Further, the laryngeal mask airway device provides ventilation of the patient's lungs without requiring contact with the sensitive inner lining of the trachea and the size of the airway established is typically significantly larger than the size of the airway established with an endotracheal tube. Also, the laryngeal mask airway device does not interfere with coughing to the same extent as endotracheal tubes. Largely due to these advantages, the laryngeal mask airway device has enjoyed increasing popularity in recent years.

Endotracheal tubes are well known in the prior art and almost invariably utilise a flexible airway tube as opposed to a rigid one. Applicant's own U.S. Patent No. 4,509,514 is the first of many publications that describe laryngeal mask airway devices and in this document it is noted that the airway tube can comprise a pre-curved rigid or flexible tube. In practice, early devices tended to employ flexible airway tubes.

Flexible airway tubes conform to the shape and in particular curvature of the patient's airway in use and as such are advantageous because they are softer and therefore more comfortable and less likely to cause damage to the patients anatomy, and further can be easily manipulated as required by a physician or nurse. In general they are also easy to insert by a trained practitioner. There are however disadvantages. Thus, where insertion must be performed by untrained personnel such as in emergency situations, and in situations where the patient has a difficult airway, correct insertion is made more difficult by the flexibility of the airway tube. In the case of a laryngeal mask device, the flexible tube cannot function effectively to direct and appropriately position the mask and often a separate additional device is required, known as an introducer, that is used to guide the device into position. Furthermore, flexible airway tubes tend to be formed from soft materials that are easily damaged, and can require the use of a rigid supporting element within the tube wall both to impart resilience and provide resistance to crushing, making manufacture more complex. To protect both flexible and also non-flexible airway tubes from damage due to patient biting, structures known as biteblocks are commonly used. The term "biteblock" has been used to describe any means that is positioned, in use, to block the patient's teeth from biting and thereby puncturing the airway and other tubes of the airway device. Biteblocks can be useful in keeping the proximal ends of the tubes of a multi-tube device together, but this can also be a disadvantage in some circumstances because the biteblock and tubes can limit access to the oral cavity of the patient.

Applicant's own WO92/13587 describes an artificial airway device that includes a rigid airway tube which is curved to follow the airway of the patient. These devices provide a solution to the problems outlined above whilst providing the additional benefit of enabling blind intubation with an endotracheal tube. However, the benefits of a flexible airway tube as outlined above are largely sacrificed.

It is an object of the invention to seek to mitigate problems such as those set out above.

According to a first aspect of the invention, there is provided an artificial airway device to facilitate lung ventilation in a patient, comprising an airway tube having a proximal end, a distal end and a biteblock, the biteblock being movable between the proximal and distal ends relative to the airway tube. The invention thus provides a device that has the well known benefits of a biteblock, but also wherein the biteblock can be moved relative to the airway tube to allow easier access to the oral cavity.

The artificial airway device may comprise a laryngeal mask airway device or an endotracheal tube. Where the device comprises a laryngeal mask airway device, it may include an airway tube and a gastric tube.

It is preferred that the biteblock is movable such that it is disposed upon or fitted to the airway tube of the device in slidable relation thereto, and it is further preferred that the biteblock and/or airway is adapted such that the position of the biteblock relative to the airway tube is maintainable by frictional force between the biteblock and the airway tube against inadvertent movement. The position of the biteblock on the airway tube can thus be adjusted to a desired position and easily maintained. It is further preferred that the biteblock is adapted to be fitted to the airway tube and movable relative thereto, by provision of a bore or channel of the biteblock into which the airway tube or other tube can fit in slidable relation thereto. It is preferred that the airway tube is resiliently deformable.

In an alternative embodiment, the biteblock may comprise a plurality of bores to accommodate some or all of the tubes of a multi-tube device, such as a laryngeal mask airway with a gastric tube or tubes. In this case, where there are multiple bores, the biteblock may comprise at least one bore that is adapted to retain a tube of a device, whilst also allowing for removal and replacement of the tube from the bore by a user. In particular, the biteblock may comprise a resiliently deformable material and at least one bore may comprise a re-entrant "C" in cross section, such that a tube may be inserted and removed by temporarily deforming the material to allow for the tube to pass through the mouth of the "C". In a multi-tube device, the ability to move one or other of the airway or gastric tubes temporarily out of position is valuable because it improves access to the oral cavity of the patient.

It is preferred that the biteblock comprises a material that is resiliently deformable, and further is relatively more rigid than the material forming the airway tube of the device on which it is intended to be used. It is still further preferred that the biteblock is formed from a silicone or plastics material.

According to a second aspect of the invention, there is provided an artificial airway device to facilitate lung ventilation in a patient, comprising a flexible airway tube having a proximal end and a distal end and a mask at the distal end, and an introducer that is connected to the device. It is preferred that the introducer comprises means adapted to releasably confer rigidity to the airway tube at or towards its distal end. The invention thus provides a device that combines the advantages of both flexible and fixed curvature airway tubes, in that it can be configured for ease of insertion and configured for comfort once inserted.

It is preferred that the device includes a fixed curve part disposed at the distal end of the airway tube. It is preferred that the fixed curve part is relatively more rigid than the airway tube. The fixed curve part may be integrally formed with or be a part of the mask, or it may be integrally formed with or a part of the airway tube. In particular the fixed curve part may comprise a connector section by which the airway tube and mask are connected.

It is preferred that the means adapted to releasably confer rigidity to the airway tube is slidable along the airway tube. This provides a simple and convenient mechanism of operation.

It is preferred that the means adapted to releasably confer rigidity to the airway tube comprises a sleeve that encircles all or a part of the circumference of the airway tube and is slidable therealong. It is further preferred that the position of the rigidity conferring means is maintained by frictional force against inadvertent movement. It is still further preferred that the means is capable of acting as a biteblock when it is appropriately positioned towards the proximal end of the airway tube by a user. It is most preferred that the means comprises a biteblock as described above.

According to one embodiment of this aspect of the invention, the device may comprise a laryngeal mask airway device or any such supraglottic airway. The laryngeal mask airway device may include an oesophageal access tube and it is preferred that the means adapted to releasably confer rigidity to the airway tube comprises a sleeve that encircles all or a part of the circumference of the airway tube and also the oesophageal access tube and is slidable along both tubes. It is preferred that the sleeve is adapted to allow release of the oesophageal tube and/or the airway tube, to facilitate manipulation of the tubes when the device is in position in a patient.

It is preferred that the flexible airway tube comprises a wall formed from a plastics material, and that the wall includes an integral supporting wire. It is preferred that the supporting wire comprises nitinol.

According to a third aspect of the invention, there is a provided a method of inserting a laryngeal mask into a patient, comprising the step of releasably increasing the rigidity of the airway tube during insertion. It is preferred that the step of increasing the rigidity of the airway tube includes deploying a releasable stiffening part of the device to a point on the airway tube at or adjacent its distal end, and it is further preferred that the releasable stiffening part is slidable, relative to the airway tube. The releasable stiffening part may also be movable to function as a biteblock when deployed in a suitable position on the airway tube.

The invention will be further described by way of example and with reference to the accompanying drawings, in which;
Figure 1 is a dorsal perspective view of a device according to the invention;
Figure 2 is a part ventral view of the device of Figure 1 in a first position;
Figure 3 is a side view of the device of Figure 2;
Figure 4 is a dorsal perspective view of the device of Figure 1 in a first position;
Figure 5 is a side view of the device of Figure 1 in a second position;
Figure 6 is a dorsal view of the device of Figure 1 in a third position; and
Figures 7a and 7b are enlarged views of a second embodiment of device according to the invention.

Referring to the drawings, there is illustrated an artificial airway device 1 to facilitate lung ventilation in a patient, comprising an airway tube 2 having a proximal end 3 and a distal end 4, a mask 5 at the distal end 4, and means 6 adapted to releasably confer rigidity to the airway tube 2 at least at, or towards its distal end 4 and also to provide a movable biteblock.

The device 1 illustrated here is of the type known as a laryngeal mask airway device. The laryngeal mask airway device includes three basic components; an airway tube 2; a backplate 7; and a cuff 8. Such devices are well known in the art, and although there are many variations in the nature of these basic components and the way that they fit together, the basic structure is common to most if not all.

The device 1 has a proximal end 9, which is the end located nearest the operator when the device is in use, and a distal end 10. At its distal end 10 the device 1 has the mask 5 and the mask 5 includes the backplate 7 and the cuff 8, as referred to above. In the illustrated embodiment, the cuff 8 is a generally elliptical inflatable ring formed from a soft material such as silicone, although it is not necessarily inflatable. The ring encircles and is attached to the backplate 7 and is configured to establish a peripherally sealed engagement of the device 1 to a patient's laryngeal inlet when the device is in place in a patient.

The backplate 7, which may be provided in the form of a bowl shape, or indeed any type of web, separates in use, the laryngeal side of the device 1 from the pharyngeal side and defines an interior hollow or lumen of the mask. The terms "laryngeal side" and "pharyngeal side" refer to the sides of the device by reference to the patient's anatomy and are as used in the art. In this embodiment the backplate 7 includes a connector section 11 that defines an aperture (not shown) through which gas may pass into the interior hollow 11a of the mask and thence to the patient, in use. The connector section 11 is basically a short tube with a roughly elliptical, or flattened cross section. Gas is passed into the interior hollow 11a via airway tube 2 which is either attached to, or formed integrally with the backplate 7. In the illustrated embodiment the connector section 11 extends in the proximal direction beyond the proximal extremity 13 of the cuff 8 and curves towards the plane of the cuff 8. Partly by virture of its shape, but also due to the material from which it is formed and its thickness, the backplate 7 is resiliently deformable but relatively rigid. It will be noted that the backplate 7 in this embodiment has a relatively low profile, a flat dorsal surface and a preformed curve that has the effect of making insertion of the device easier.

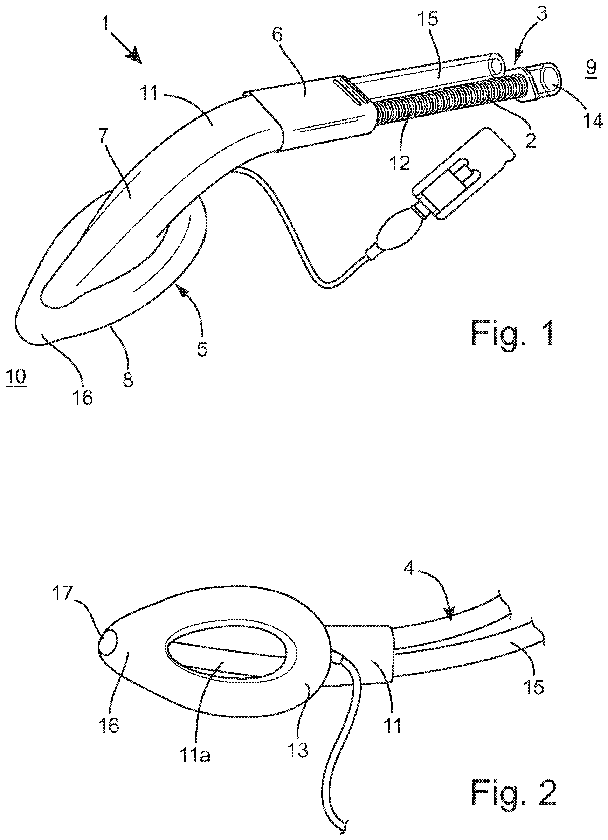

The airway tube 2 of the device 1 in this embodiment is a generally straight tube formed from a soft plastics or silicone material. The tube includes a lumen that extends longitudinally from one end to the other. The tube does not have a preformed curvature, but by virtue of its softness it can be easily bent into a curve as desired and by virtue of its resilient deformability, will tend to return to its unbent state when released. The airway tube 2 is thus relatively less rigid than the mask 5. Due to the softness of the tube 2 its wall can be supported by an integrally disposed support 12 (Figure 1), which in this embodiment takes the form of a nitinol wire which extends in close spirals around the circumference of the tube 2 for its entire length, as is known in the art, and helps prevent occlusion of the lumen by kinking. As already described, the airway tube 2 has a proximal end 3 and a distal end 4. The proximal end 3 of the airway tube includes a removable tubular connector 14 to allow the airway tube 2 to be connected to a supply of gas. The distal end 4 of the airway tube 2 passes into the connector section 11 of the backplate 7 and is sealed therein such that the lumen of the airway tube is in fluid communication with the aperture of the connector section 11.

As will be appreciated from the figures, the device 1 illustrated in this example is of the type of laryngeal mask airway that includes a gastric tube. A gastric tube is now a well-known addition to laryngeal mask airways and provides for the safe evacuation of regurgitated or vomited material from a patient's oesophagus that might otherwise be at risk of entering the patient's lungs should the seal of the cuff 8 around the laryngeal inlet be compromised. It is very important that this does not occur because it can lead to serious lung damage. A gastric tube is thus a desirable addition to most types of laryngeal mask airway where it can be accommodated, but its inclusion can lead to undesirable consequences in terms of the physical properties of the resultant device, which manifest themselves in difficulty of insertion and reduced effectiveness of the primary function as an airway. In the illustrated embodiment, the gastric tube 15 comprises a soft, straight, circular section tube of plastics or silicone material. The gastric tube 15 passes into the connector section 11 of the backplate 7, extends across the interior (laryngeal side) of the bowl and crosses longitudinally within the distal tip 16 of the cuff 8 in sealed engagement therewith and defining at its terminal extent a gastric aperture 17 in the cuff wall. This type of construction is well known in the art, in Applicant's own "Proseal" device for example, and is an example only of how a gastric tube may be provided. There are many others, all of which would be suitable for use with the present invention with suitable modification. It will be understood that a gastric tube is not an essential element of the present invention.

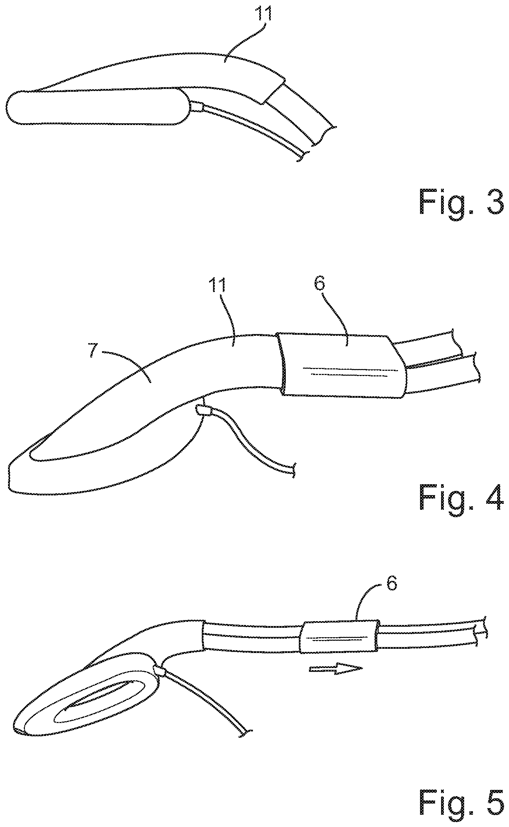

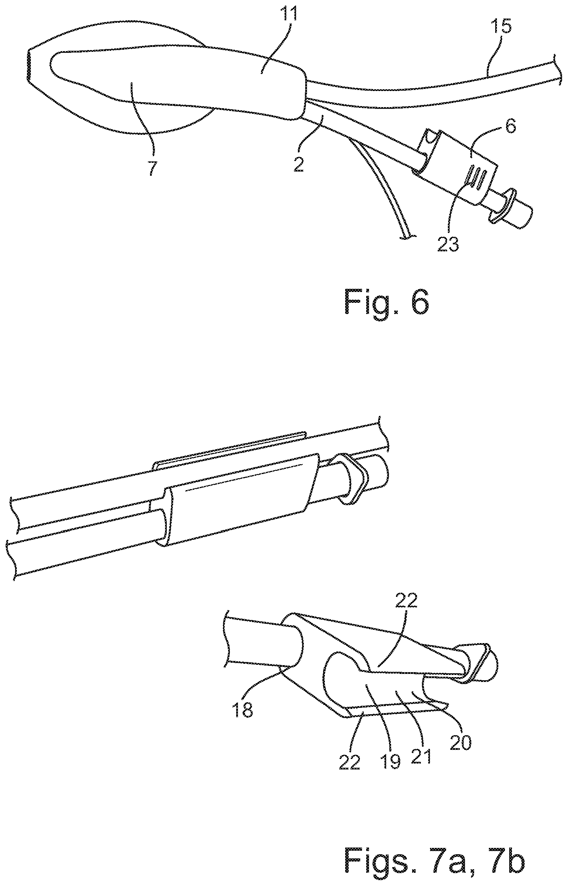

Referring particularly to Figures 4 and 5, it can be seen that the device 1 is provided with means 6 adapted to releasably confer rigidity to the airway tube 2 at least at, or towards, its distal end 4, and in addition, provides a movable biteblock. The said means 6 takes the form of a substantially rectangular block of relatively rigid plastics or silicone material. In terms of rigidity, the block is substantially as rigid as the connector section 11 of the backplate 7; in terms of dimensions of width and height, the block 6 is sized to extend the minimum distances possible beyond the total diameters of the two tubes so as to avoid adding unnecessary bulk to the construction; and in terms of length, the block 6 is dimensioned at a minimum to allow for easy manipulation by a user's fingers. The block 6 includes two parallel, longitudinally extending open bores 18, 19 (Figures 7a, 7b) formed integrally within it and sized, in terms of internal diameter, to receive there through in snug but slidable relation one each of the airway tube 2 and gastric tube 15. Thus, there is an airway tube bore 18 and a gastric tube bore 19. The bores 18, 19 differ, in that the gastric tube bore 19 is open along its entire length thus forming a channel 20. The channel wall is a re-entrant "C" in cross-section and thus the gap or opening 21 is defined by parallel opposing lips 22 of the mouth of the "C", the distance between which is narrower than the diameter of the gastric tube 15. As a result, in order to insert the gastric tube 15 into the bore 19 the lips 22 must be forced apart, a movement that is facilitated by the resilient deformability of the material from which they are formed. Once the gastric tube 15 has passed the lips 22 into the bore 19 the lips 22 will spring back into position thus retaining the gastric tube 15 in position in the manner of a mechanical lock, whilst still allowing it to slide therein. The dimensions of the bores 18, 19 are selected by reference to the dimensions of the airway and gastric tubes such that the block 6 can be slid along the tubes relatively freely under manual force, but that once in a desired position it will remain there against inadvertent movement through friction between the surfaces of the bores and the tubes. On its dorsal and ventral surfaces the block 6 is provided with friction ridges 23 to aid in sliding.

In use, the device 1 is inserted into a patient by a user, mask-end first in the usual manner. However, unlike with existing devices that utilise flexible airway tubes where a user is required to press with a finger on the proximal end of the mask, with the device 1 the user simply slides the block 6 in the distal direction along the airway tube to a first or insertion position or station in which the block 6 is adjacent to and preferably butting up against the backplate 7 (Figures 1, 4). When the block 6 is in that position it will be readily appreciated that it has the effect of stiffening the airway tube 2 along a length equal to the longitudinal extent of the block 6 by preventing the tube 2 from bending or kinking and also provides a rigid structure that impinges on the similarly rigid backplate 7 allowing insertion force to be appropriately transmitted without unwanted bending. Once the insertion manoeuvre is complete, the block 6 is then conveniently slid back in the proximal direction to a second position in which the flexibility of the airway tube is unaffected. It can thus be said that the device 1 includes an integral or built-in introducer.

In the illustrated embodiment, block 6 is configured such that it is also able to function as a biteblock when moved into an appropriate position between the patient's teeth. As will be appreciated, this position will vary from patient to patient, and thus the device 1 has the added advantage of allowing for a bespoke fit. In terms of rigidity, the block is provided to be sufficiently rigid to withstand biting, but also sufficiently soft and resilient to avoid damage to the teeth if biting should occur.

The biteblock 6 may be applied to any airway device that includes an airway tube with no protection against biting by a patient. As will be appreciated, the biteblock 6 can be attached to the airway tube 2 by simply sliding it thereon from the free end and advancing it to the desired position as dictated by the patient's particular anatomy to achieve a bespoke fit. The biteblock 6 can be moved relative to the airway tube 2 to increase access to the oral cavity of the patient when that is required and afterwards moved back into position to protect the tube 2. In addition, the gastric tube can be removed from the block 6 all together (Figures 6, 7a, 7b) by releasing it sideways, as viewed, from the re-entrant "C", to further improve access to the oral cavity of the patient, the block 6 being positionable as desired on the airway tube 2.

## Claims

1. An artificial airway device (1) to facilitate lung ventilation in a patient, comprising a flexible airway tube (2) having a proximal end (3) and a distal end (4) and a mask (5) at the distal end, **characterised by** an introducer (6) that is connected to the device, wherein the introducer comprises means adapted to releasably confer rigidity to the airway tube at or adjacent its distal end that is slidable along the airway tube, and wherein the rigidity conferring means is adapted to act as a bite block when it is positioned towards the proximal end of the airway tube by a user.

2. A device according to claim 1, wherein the means adapted to releasably confer rigidity to the airway tube comprises a sleeve that encircles all or a part of the circumference of the airway tube and is slidable therealong.

3. A device according to claim 1 or claim 2, wherein the position of the rigidity conferring means is maintainable against movement by frictional force.

4. A device according to any of claims 1 to 3, comprising a laryngeal mask airway device.

5. A device according to any of claim 4, further including an oesophageal tube.

6. A device according to claim 5, wherein the means adapted to releasably confer rigidity to the airway tube comprises a sleeve that encircles all or a part of the circumference of the airway tube and also the oesophageal tube and is slidable along both tubes.

7. A device according to claim 6, wherein the sleeve is adapted to allow release of the oesophageal tube and/or the airway tube, to facilitate manipulation of the tubes when the device is in position in a patient.

8. A device according to any preceding claim, wherein the flexible airway tube comprises a wall formed from a plastics material, and includes an integral supporting wire.

9. A device according to claim 8, wherein the wire comprises nitinol.

## Patentansprüche

1. Künstliche Luftwegvorrichtung (1) zum Erleichtern der Lungenbeatmung bei einem Patienten, umfassend einen biegsamen Luftwegtubus (2) mit einem proximalen Ende (3) und einem distalen Ende (4) und einer Maske (5) an dem distalen Ende, **gekennzeichnet durch** eine Einführeinrichtung (6), die mit der Vorrichtung verbunden ist, wobei die Einführeinrichtung ein Mittel umfasst, das dazu angepasst ist, dem Luftwegtubus an oder benachbart seinem distalen Ende lösbar Steifheit zu verleihen, und das entlang dem Luftwegtubus verschiebbar ist, und wobei das Steifheitsverleihungsmittel dazu angepasst ist, als Beißklotz zu wirken, wenn es von einem Benutzer zum proximalen Ende des Luftwegtubus hin positioniert wird.

2. Vorrichtung nach Anspruch 1, wobei das Mittel, das dazu angepasst ist, dem Luftwegtubus lösbar Steifheit zu verleihen, eine Hülse umfasst, die den Umfang des Luftwegtubus ganz oder teilweise umkreist und daran entlang verschiebbar ist.

3. Vorrichtung nach Anspruch 1 oder Anspruch 2, wobei die Lage des Steifheitverleihungsmittels durch Reibkraft Bewegung entgegen gehalten werden kann.

4. Vorrichtung nach einem der Ansprüche 1 bis 3, umfassend eine Kehlkopfmasken-Luftwegvorrichtung.

5. Vorrichtung nach Anspruch 4, weiter umfassend einen Speiseröhrentubus.

6. Vorrichtung nach Anspruch 5, wobei das Mittel, das dazu angepasst ist, dem Luftwegtubus lösbar Steifheit zu verleihen, eine Hülse umfasst, die den Umfang des Luftwegtubus und außerdem des Speiseröhrentubus ganz oder teilweise umkreist und entlang beider Röhren verschiebbar ist.

7. Vorrichtung nach Anspruch 6, wobei die Hülse dazu angepasst ist, das Lösen des Speiseröhrentubus und/oder des Luftwegtubus zu ermöglichen, um die Manipulation der Tubusse zu erleichtern, wenn sich die Vorrichtung in einem Patienten in Position befindet.

8. Vorrichtung nach einem der vorangehenden Ansprüche, wobei der biegsame Luftwegtubus eine aus einem Kunststoff gebildete Wand umfasst und einen integrierten Stützdraht umfasst.

9. Vorrichtung nach Anspruch 8, wobei der Draht Nitinol umfasst.

## Revendications

1. Dispositif de voie aérienne artificiel (1) pour faciliter la ventilation pulmonaire chez un patient, comprenant un tube de voie aérienne flexible (2) ayant une extrémité proximale (3) et une extrémité distale (4) et un masque (5) au niveau de l'extrémité distale, **caractérisé par** un introducteur (6) qui est connecté au dispositif, dans lequel l'introducteur comprend un moyen conçu pour transmettre de la rigidité de manière libérable au tube de voie aérienne au niveau ou adjacent à son extrémité distale qui peut coulisser le long du tube de voie aérienne, et dans lequel le moyen de transmission de rigidité est conçu pour agir comme un bloc à mordre quand il est positionné vers l'extrémité proximale du tube de voie aérienne par un utilisateur.

2. Dispositif selon la revendication 1, dans lequel le moyen conçu pour transmettre de la rigidité de manière libérable au tube de voie aérienne comprend un manchon qui encercle l'ensemble ou une partie de la circonférence du tube de voie aérienne et peut coulisser le long de celui-ci.

3. Dispositif selon la revendication 1 ou la revendication 2, dans lequel la position du moyen de transmission de rigidité peut être maintenu contre tout mouvement par la force de frottement.

4. Dispositif selon l'une quelconque des revendications 1 à 3, comprenant un dispositif de voie aérienne à masque laryngé.

5. Dispositif selon la revendication 4, incluant en outre un tube oesophagien.

6. Dispositif selon la revendication 5, dans lequel le moyen conçu pour transmettre de la rigidité de manière libérable au tube de voie aérienne comprend un manchon qui encercle l'ensemble ou une partie de la circonférence du tube de voie aérienne et aussi du tube oesophagien et peut coulisser le long des deux tubes.

7. Dispositif selon la revendication 6, dans lequel le manchon est conçu pour permettre la libération du tube oesophagien et/ou du tube de voie aérienne, pour faciliter la manipulation des tubes quand le dispositif est en position dans un patient.

8. Dispositif selon l'une quelconque des revendications précédentes, dans lequel le tube de voie aérienne flexible comprend une paroi formée d'une matière plastique, et inclut un fil de support intégral.

9. Dispositif selon la revendication 8, dans lequel le fil comprend du nitinol.
